(19) Europäisches Patentamt
European Patent Office
Office européen des brevets

(11) Publication number: 0 154 894
B1

(12) **EUROPEAN PATENT SPECIFICATION**

(45) Date of publication of patent specification: 20.04.88

(51) Int. Cl.[4]: C 07 C 149/16, C 07 C 149/18, C 07 C 149/31, C 07 C 149/34, C 07 C 149/36

(21) Application number: 85102199.8

(22) Date of filing: 27.02.85

# (54) A sulfur-containing fluorinated unsaturated compound and its manufacturing process.

(30) Priority: 29.02.84 JP 38380/84

(43) Date of publication of application: 18.09.85 Bulletin 85/38

(45) Publication of the grant of the patent: 20.04.88 Bulletin 88/16

(84) Designated Contracting States: CH DE FR GB LI

(56) References cited:
DD-A- 204 252
GB-A-2 058 763

(73) Proprietor: DAIKIN INDUSTRIES, LIMITED
Shinhankyu-Building, 12-39, I-chome Umeda
Kita-ku Osaka-shi Osaka (JP)

(72) Inventor: Ishikawa, Nobuo, Prof. Dr.-Ing.
10-10, Shinoharadai-machi Kohoku-ku
Yokohama-shi Kanagawa-ken (JP)
Inventor: Kitazume, Tomoya, Dr.-Ing.
24-1-115, Kitasenzoku 3-chome
Ota-ku Tokyo (JP)

(74) Representative: Patentanwälte TER MEER - MÜLLER - STEINMEISTER
Mauerkircherstrasse 45
D-8000 München 80 (DE)

EP 0 154 894 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a sulfur-containing fluorinated unsaturated compound and a process for its manufacture.

In recent years, fluorinated organic compounds have attracted much public attention in various fields for their use as medicines, pesticides, various surface treatment agents etc. An important problem with these compounds is how to selectively introduce a fluorine atom or atoms at target sites on the molecule. However, the method that readily allows such selective introduction of fluorine atoms in the molecule in high yields has so far been reported rather rarely. Namely, there are only very few disclosures of such method, for example, the one referring to an α-trifluoromethyl malonic acid ester published at the 8th Fluorine Chemistry Symposium in Japan (1982). This invention is primarily intended to solve such problem.

Accordingly, it is an object of the present invention to provide an α,β-unsaturated carbonyl compound or carbinol carrying a fluorine atom or atoms at target sites of its molecule and useful as a building block to allow introduction of a fluorine atom or atoms in the molecule of other compounds and a process for its manufacture.

The invention therefore relates to a sulfur-containing unsaturated compound having the general formula

$$R^1-\overset{\overset{\displaystyle O}{\|}}{C}-CF=CF-S-R^2$$

wherein $R^1$ and $R^2$ are independently alkyl groups having up to eight carbon atoms with or without a substituent or substituents and $R^2$ can also be a phenyl group with or without a substituent or substituents and a sulfur-containing fluorinated unsaturated compound having the general formula

$$R^1-\underset{\underset{\displaystyle R^3}{|}}{\overset{\overset{\displaystyle OH}{|}}{C}}-CF=CF-S-R^2$$

wherein $R^1$, $R^2$ and $R^3$ are independently alkyl groups having up to eight carbon atoms with or without a substituent or substituents and $R^2$ and $R^3$ can also be phenyl groups with or without a substituent or substituents.

A further subject matter of the invention is a process for the manufacture of such sulfur-containing fluorinated α,β-unsaturated carbonyl compounds in high yields, which is characterized in that a halide of a fluorinated carbonyl compound having the general formula

$$R^1-\overset{\overset{\displaystyle O}{\|}}{C}-CHF-CF_2X$$

wherein $R^1$ is an alkyl group having up to eight carbon atoms with or without a substituent or substituents and X is a halogen atom, is reacted with a mercaptan having the general formula

$$R^2-SH$$

wherein $R^2$ is an alkyl group having up to eight carbon atoms or a phenyl group each with or without a substituent or substituents, to produce a sulfur-containing fluorinated carbonyl compound having the general formula

$$R^1-\overset{\overset{\displaystyle O}{\|}}{C}-CHF-CF_2-S-R^2$$

wherein $R^1$ and $R^2$ are the same as those of the starting compounds, and said sulfur-containing fluorinated carbonyl compound is converted, by the elimination of hydrogen fluoride, to the corresponding sulfur-containing fluorinated unsaturated carbonyl compound having the general formula

$$R^1-\overset{\overset{\displaystyle O}{\|}}{C}-CF=CF-S-R^2$$

wherein $R^1$ and $R^2$ are the same as those of the starting compounds.

Groups $R^1$, $R^2$ and $R^3$ in the above general formulae of the sulfur-containing fluorinated unsaturated compounds of the invention are independently alkyl groups having up to eight carbon atoms, preferably straight chain alkyl groups, or phenyl groups with or without a substituent or substituents. Examples of said alkyl groups are methyl, ethyl, propyl, isopropyl, butyl or isobutyl groups. Groups $R^1$, $R^2$ and $R^3$ may be substituted for example with a halogen atom or atoms.

The above sulfur-containing fluorinated α,β-unsaturated carbonyl compound is useful to manufacture α - fluoro - α,β - unsaturated carboxylic acid thioesters, β-fluorallyl alcohols, etc. which are useful as building block of monofluorinated compounds.

Moreover, the definition of $R^1$ and $R^2$ as mentioned above also applies groups $R^1$ and $R^2$ in the general formulae of the halide of fluorinated carbonyl compound and the mercaptan that are used in the above process of the invention to prepare the above unsaturated carbonyl compound. The reaction of such halide and mercaptan, if made under presence of base such as triethylamine, can produce the sulfur-containing fluorinated carbonyl compound as mentioned above in high yield (for example, 96% for the isolated compound).

To convert such carbonyl compound to the intended sulfur-containing fluorinated α,β-unsaturated carbonyl compound by the elimination of hydrogen fluoride, it is preferable to conduct the reaction under the action of a base such as triethylamine in an alcoholic solvent, for example, methanol, ethanol, propanol or isopropanol.

This is probably because the fluoride ion $F^-$, as it is eliminated, is stabilized by solvation with the alcoholic hydroxyl group to facilitate further elimination of hydrogen fluoride. To the contrary, it has been confirmed that if the reaction proceeds under the action of triethylamine in an aprotic polar solvent such as dimethylformamide, no hydrogen fluoride is eliminated and that if the base solution is aqueous solution of sodium hydroxide or sodium bicarbonate, the corresponding unsaturated carbonyl compound is produced only in low yield probably because it is hydrolyzed in further reaction to a β-ketocarboxylic acid.

The invention will be described below with reference to concrete examples.

First, the trifluoroethene is acylated by a Friedel-Crafts reaction to 4 - chloro - 3,4,4 - trifluoro - 2 - butanone *1* ($CH_3C(O)CHFCF_2Cl$) according to the following process of prior art:

$$CF_2{=}CHF \xrightarrow[AlCl_3]{CH_3C(O)Cl} CH_3C(O)CHFCF_2Cl$$

*1*

Butanone *1* is reacted with thiophenol(PhSH)*2* in dichloroethane in the presence of triethylamine to produce an adduct 4 - phenylthio - 3,4,4 - trifluoro - 2 - butanone *3* in the following reaction:

$$CH_3C(O)CHFCF_2Cl + PhSH$$

*1* *2*

$$\xrightarrow[-78°C\ 0.5\ hr]{(C_2H_5)_3N/CH_2Cl_2} CH_3C(O)CHFCF_2SPh$$

*3*

(Isolation yield: 96%)

+

$$CH_3C(O)CF{=}CFSPh$$

*4*

Actually, *1* in the above reaction is first converted to an intermediate product *1a* in the following reaction. Quick addition of PhSH to *1a* then produces *3* while elimination of hydrogen fluoride gives a by-product *4*.

$$CH_3C(O)CHFCF_2Cl \xrightarrow{-HCl} CH_3C(O)CF{=}CF_2$$

*1* *1a*

$$1a + PhSH \rightarrow 3 + 4$$

The above reaction can be expressed by the following general formula:

$$R^1C(O)CHFCF_2Cl + R^2SH$$

*1*

$$\xrightarrow{(C_2H_5)_3N/CH_2Cl_2} R^1C(O)CHFCF_2SR^2$$

*3*

$$+$$

$$R^1C(O)CF{=}CFSR^2$$

*4*

Examples of adduct *3* prepared by the above reaction are listed in the following table:

TABLE 1

| $R^1$ | $R^2SH$ | Yield of *3* (%) | Ratio (*3/4*) | E/Z Ratio of *4* | B.p. of *3* (°C/mmHg) |
|---|---|---|---|---|---|
| $CH_3$— | PhSH | 96 | *3*>99% | — | 78~80/0.6 |
| $C_2H_5$— | PhSH | 90 | *3*>99% | — | 95~96/0.7 |
| $CH_3$— | $C_2H_5SH$ | 80 | 44/56 | 33/67 | —* |

* No isolation.

NMR and IR spectral data of these adducts are given in Table 2.

Next, the above adduct *3* is reacted with triethylamine in alcoholic solvent, for example, isopropanol for the synthesis of 2 - phenylthio - 1,2 - difluoroethenylketone *4′*.

TABLE 2

| *3* or *4* | NMR: Chem. shift (δ ppm) $^1$H (Solv: $CCl_4$) | NMR: Chem. shift (δ ppm) $^{19}$F (No solv.) | NMR: Coupling const. J (Hz) | IR $v_c$=0 ($cm^{-1}$) |
|---|---|---|---|---|
| $CH_3^aC(O)CH^bF^{a'}$—<br>$CF_2^{b'c'}SC_6H_5^c$ | a) 2.28 (d)<br>b) 4.65 (d, d, d)<br>c) 7.2~7.7 | a′) 115.4 (d, d, d, q)<br>b′) 3.4 (d, d, d)<br>c′) 6.2 (d, d, d) | Jaa′=4.5, Ja′b′=19.8<br>Jba′=46.9, Ja′c′=17.3<br>Jbb′=8.7, Jb′c′=216.8<br>Jbc′=12.6 | 1735 |
| $CH_3^aCH_2^bC(O)$—<br>$CH^cF^{a'}CF_2^{b'c'}SC_6H_5^d$ | a) 1.03 (t)<br>b) 2.62 (d, q)<br>c) 4.73 (d, d, d)<br>d) 7.2~7.8 | a′) 116.3 (d, d, d, t)<br>b′) 3.4 (d, d, d)<br>c′) 6.6 (d, d, d) | Jab=7.4, Ja′b′=20.2<br>Jba′=2.4, Ja′c′=17.0<br>Jca′=47.0, Jb′c′=216.0<br>Jcb′=8.7<br>Jcc′=12.2 | 1735 |
| $CH_3^aC(O)CH^bF^{a'}CF_2^{b'c'}SCH_2^cCH_3^d$<br><br>$CH_3^gC(O)CF^{d'}{=}CF^{e'}SCH_2^eCH_3^f$ | a) 2.30 (d)<br>b) 4.84 (d, d, d)<br>c) 2.93 (q)<br>d) 1.38 (t)<br>e) 3.00 (q)<br>f) 1.43 (t)<br>g) 2.31 (d, d) | a′) 117.0 (d, d, d, q)<br>b′) 7.5 (d, d, d)<br>c′) 6.1 (d, d, d)<br>E {d′) 66.5 (q); e′) 24.6 (s)}<br>Z {d′) 74.2 (d, q); e′) 34.4 (d, q)} | Jaa′=4.5, Ja′b′=16.9<br>Jba′=48.6, Ja′c′=19.2<br>Jbb′=10.5, Jb′c′=226.2<br>Jbc′=9.0<br>Jcd=Jef=7.5<br>E {Jgd′=4.5; Jd′e′=0}<br>Z {Jgd′=Jge′=4.5; Jd′e′=135.5} | 1735 |

$CH_3C(O)CHFCF_2SPh$

*3*

$(C_2H_5)_3N$/Isopropanol

10°C, 0.5 hr

$CH_3C(O)CF{=}CFSPh$

*4'*

The compound *4'* has the same structure as the compound *4* as mentioned above. While the *3*, as it is synthesized, is partially converted to the *4* by elimination of hydrogen fluoride, the remaining *3* is further converted to the *4'* by efficient elimination of hydrogen fluoride.

The above hydrogen fluoride elimination can be expressed by the following general formula:

$R^1C(O)CHFCF_2SR^2$

*3*

$(C_2H_5)_3N$/Isopropanol

$R^1C(O)CF{=}CFSR^2$

*4'*

A likely reason why there occurs sufficient elimination of hydrogen fluoride is that the fluoride anion $F^-$, as it is freed from the *3*, might be stabilized by solvation with the alcoholic hydroxyl group resulting in facilitation of hydrogen fluoride elimination. Also when methanol is used for the alcohol, the *4'* is produced in high yield.

Examples of the vinyl ketone *4'* prepared by the above reaction are listed in the following table:

TABLE 3

| $R^1$ | $R^2$ | Isolation yield (%) | E/Z ratio | B.p. (°C/mmHg) |
|---|---|---|---|---|
| $CH_3$— | Ph— | 96 | 25/75 | 81~95/0.2 |
| $C_2H_5$— | Ph— | 98 | 30/70 | 90~105/0.5 |

It is noted that the above sequence of reaction steps to synthesize the *4* (or *4'*) from the *1* can be conducted in a one-pot process, for example, using *1* and *2* in an equivalent amount.

The above ketone *4* (or *4'*) can be used to derive various fluorinated organic compounds as described below.

First, this ketone reacts with a Grignard reagent ($R^3MgX$) according to the following reaction to produce the corresponding carbinol *5* in high yield.

$R^1C(O)CF{=}CFSR^2 + R^3MgX$

*4*

0°C, 30 min

$H_3O^+$

Satd. $NH_4Cl$

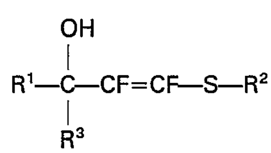

*5*

Carbinols *5* thus obtained are listed in the following table.

TABLE 4

| $R^1$ | $R^2$ | $R^3$ | Solvent | Yield* of *5*(%) |
|---|---|---|---|---|
| $CH_3$— | Ph— | $CH_3$— | $(C_2H_5)_2O$ | 98 |
| $CH_3$— | Ph— | $C_2H_5$— | $(C_2H_5)_2O$ | 98 |
| $CH_3$— | Ph— | $C_2H_5$— | Tetrahydrofuran | 48 |
| $CH_3$— | Ph— | i-$C_3H_7$— | $(C_2H_5)_2O$ | 58 |
| $CH_3$— | Ph— | Ph— | Tetrahydrofuran | 96 |
| $C_2H_5$— | Ph— | $CH_3$— | $(C_2H_5)_2O$ | 98 |
| $C_2H_5$— | Ph— | $C_2H_5$— | $(C_2H_5)_2O$ | 98 |
| $C_2H_5$— | Ph— | Ph— | Tetrahydrofuran | 98 |

* $^{19}F$ NMR yield (external standard: $PhCF_3$).

Further, the ketone *4* is reduced by sodium boron hydride to the carbinol *5′* in high yield according to the following reaction ($R^3$=H):

$R^1C(O)CF=CFSR^2$

*4*

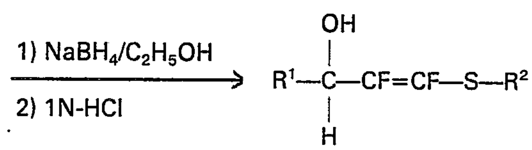


*5′*

Carbinols *5′* thus obtained by the above reaction are listed in the following table:

TABLE 5

| $R^1$ | $R^2$ | Yield (%) Isolation | Yield (%) $^{19}F$ NMR |
|---|---|---|---|
| $CH_3$— | Ph— | 95 | >98 |
| $C_2H_5$— | Ph— | 90 | >98 |

Various carbinols (*5* and *5'*) obtained by the above reactions gave analytical data as summarized in Tables 6 and 7.

TABLE 6

| $OH^a$<br>\|<br>$R^1R^3CCF^{a'}=CF^{b'}SC_6H_5^b$ | NMR: Chem. shift (δ ppm) $^1H$ ($CCl_4$) | NMR: Chem. shift (δ ppm) $^{19}F$ (no solv.) | NMR: Coupling const. (Hz) |
|---|---|---|---|
| $R^1=R^3=CH_3^c$ | a) 2.5~3.0 (br)<br>b) 7.1~7.5<br>c) 1.49 (d) | E: a') 37.6 (d, sep)<br>b') 33.6 (d)<br>Z: a') 54.6 (d, sep)<br>b') 49.4 (d, sep) | E: Jca'=2<br>Ja'b'=5<br>Z: Jca'=2<br>Jcb'=3<br>Ja'b'=143 |
| $R^1=CH_3^c$,<br>$R^3=CH_3^dCH_2^e$ | a) 2.6 (Z), 3.1 (E)<br>b) 7.1~7.5<br>c) 1.43 (d, d)<br>d) 0.92 (t)<br>e) 1.72 (q) | E: a') 36.4 (br)<br>b') 32.0 (d)<br>Z: a') 53.3 (b, d)<br>b') 49.0 (d) | Jde=7.5<br>E:Ja'b'=6<br>Z:Ja'b'=143 |
| $R^1=CH_3^c$,<br>$R^3=C_6H_5^d$ | a) 2.9 (Z), 3.5 (E)<br>b) 7.1~7.5<br>c) 1.70 (d)<br>d) 7.20 (s) | E: a') 35.3 (d, q)<br>b') 33.2 (d)<br>Z: a') 52.1 (d, q)<br>b') 47.4 (d, q) | E: Jca'=3<br>Ja'b'=6<br>Z: Jca'=2<br>Jcb'=4<br>Ja'b'=141 |
| $R^1=CH_3^c$,<br>$R^3=(CH_3^d)_2CH^e$ | a) 2.6 (br)<br>b) 7.0~7.6<br>c) 1.36 (d)<br>d) 0.93 (d)<br>e) 1.94 (sep) | E: a') 34.7 (br)<br>b') 32.9 (d)<br>Z: a') 51.4 (d)<br>b') 49.2 (d, q) | Jde=7.4<br>E: Jca'=3<br>Ja'b'=5<br>Z: Jca'=3<br>Jcb'=3<br>Ja'b'=149 |
| $R^1=CH_3^cCH_2^d$,<br>$R^3=C_6H_5^e$ | a) 3.0 (Z), 3.7 (E)<br>b) 7.1~7.5<br>c) 0.90 (t), 0.94 (t)<br>d) 2.03 (d, q)<br>e) 7.20 (s) | E: a') 33.4 (br)<br>b') 32.4 (d)<br>Z: a') 51.4 (d)<br>b') 47.7 (d) | Jcd=7.5<br>E:Ja'b'=5<br>Z:Ja'b'=154 |
| $R^1=R^3=CH_3^cCH_2^d$ | a) 2.2 (Z), 3.1 (E)<br>b) 7.1~7.5<br>c) 0.95 (t)<br>d) 1.69 (d, q) | E: a') 37.7 (br)<br>b') 30.7 (d)<br>Z: a') 52.4 (d)<br>b') 51.2 (d) | Jcd=7.5<br>E:Ja'b'=5<br>Z: Jda'=4<br>Ja'b'=142 |

TABLE 7

| $R^1CH^a(OH^b)CF^{a'}=CF^{b'}SC_6H_5^c$ | NMR | | |
|---|---|---|---|
| | Chem. shift (δ ppm) | | Coupling const. (Hz) |
| | $^1H$ (solv: $CCl_4$) | $^{19}F$ (no solv.) | |
| $R^1=CH_3^d$ | a) 4.89 (d, d, q)<br>b) 2.7 (s)<br>c) 7.1~7.5<br>d) 1.36 (d) | E {a') 54.3 (d, d); b') 39.3 (d, d)}<br>Z {a') 68.1 (d, d); b') 53.8 (d, d)} | Jad=6.8<br>E {Jaa'=25; Jab'=3; Ja'b'=8}<br>Z {Jaa'=25; Jab'=4; Ja'b'=139} |
| $R^1=CH_3^dCH_2^e$ | a) 4.60 (d, d, t)<br>b) 4.2 (s)<br>c) 7.1~7.5<br>d) 0.91 (t), 0.95 (t)<br>e) 1.70 (d, q) | E {a') 53.7 (d, d); b') 36.3 (d, d)}<br>Z {a') 67.5 (d, d); b') 52.2 (d, d)} | Jad≃Jde=7.5<br>E {Jaa'=25; Jab'=3; Ja'b'=9}<br>Z {Jaa'=25; Jab'=4; Ja'b'=137} |

The above carbinols *5* and *5'* may be used for the synthesis of thioesters of α - fluoro - α,β - unsaturated carboxylic acids. Namely, when heated at 100 to 200°C (for example, between 130 and 150°C) or under addition of a catalytic amount of mineral acid (for example, conc. sulfuric acid), the carbinol *5* or *5'* is converted to the corresponding α - fluoro - α,β - unsaturated carboxylic acid thioester *6* together with the by-product *7* according to the following reaction:

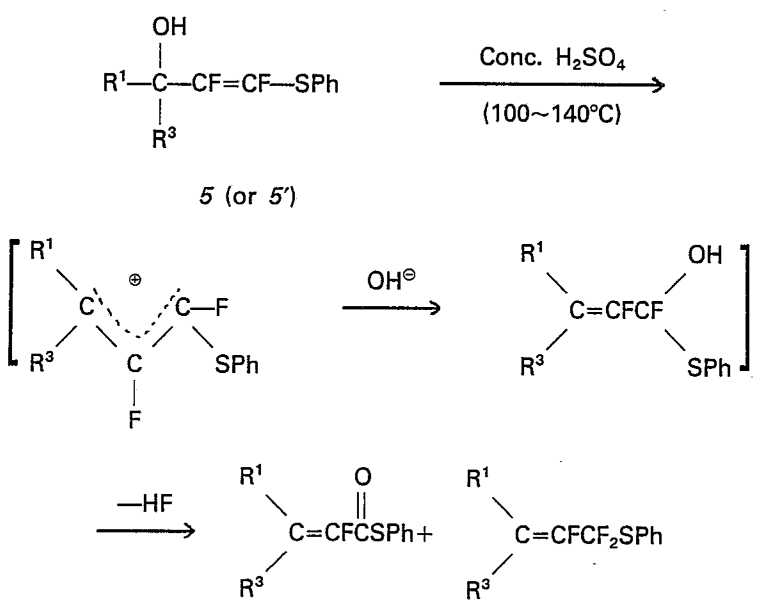


*5* (or *5'*)

*6* *7*

Various thioesters *6* and by-products *7* thus obtained are summarized in Table 8.

Further, analytical data of these thioesters *6* and by-products *7* are summarized in Tables 9 and 9', respectively.

Reduction of the above thioester *6* with sodium boron hydride readily gives the corresponding allyl alcohol *8* according to the following reaction:

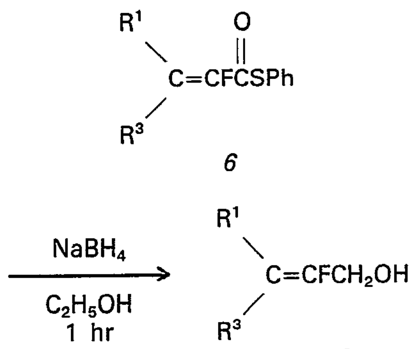


6

8

TABLE 8

| $R^1$ | $R^3$ | Reaction condition | Yield* (5) 6 | 7 | E/Z Ratio of *6* |
|---|---|---|---|---|---|
| $CH_3$— | H— | Conc. $H_2SO_4$ added | 82 (89) | Trace | <2/>98 |
| $CH_3$— | $CH_3$— | Heating | 77 (82) | 8 (10) | — |
| $CH_3$— | $C_2H_5$— | Heating | 85 (95) | Trace | 31/69 |
| $CH_3$— | $C_2H_5$— | Conc. $H_2SO_4$ added | 70 (75) | Trace | 39/61 |
| $CH_3$— | i-$C_3H_7$— | Heating | 80 (95) | Trace | 26/74 |
| $Ch_3$— | Ph— | Heating | 64 (79) | 15 (21) | 31/69 |
| $C_2H_5$— | H— | Conc. $H_2SO_4$ added | 63 (70) | Trace | <2/>98 |
| $C_2H_5$— | $C_2H_5$— | Heating | 69 (75) | 10 (20) | — |
| $C_2H_5$— | Ph— | Heating | 55 (71) | 10 (26) | 48/52 |

* Isolation yield; $^{19}F$ NMR in parentheses (external standard: $PhCF_3$).

Various allyl alcohols *8* thus obtained and some NMR and IR spectral data are summarized in Tables 10 and 11.

TABLE 9

| $R^1R^3C{=}CFC(O)SC_6H_5^a$ | | NMR | | | IR | |
|---|---|---|---|---|---|---|
| | | Chem. shift (δ ppm) | | Coupling const. (Hz) | $\nu_{c=0}$ ($cm^{-1}$)<br>($\nu_{c=c}$ ($cm^{-1}$)) | B.p. (°C) |
| $R^1$ | $R^3$ | $^1H$ (solv.: $CCl_4$) | $^{19}F$ (—) | | | |
| $CH_3^b$ | $H^c$(Z) | a) 7.40 (s)<br>b) 1.78 (d, d)<br>c) 6.03 (d, d) | 51.2 (d, q) | Jbc=7.5<br>$Jb_F$=3.0<br>$Jc_F$=34.0 | 1685<br>(1660) | |
| $CH_3^b$ | $CH_3^c$ | a) 7.37 (s)<br>b) 1.80 (d)<br>c) 2.01 (d) | 48.2 (q, q) | $Jb_F$=4.2<br>$Jc_F$=3.3 | 1685<br>(1650) | |
| $CH_3^b$ | $CH_3^cCH_2^d$ | E { a) 7.40 (s)<br>b) 1.81 (d)<br>c) 0.99 (t)<br>d) 2.46 (d, q) }<br>Z { b) 2.00 (d)<br>c) 1.04 (t)<br>d) 2.21 (d, q) } | 48.3 (t, q)<br>49.7 (t, q) | $Jb_F$=4.5<br>$Jd_F$=1.7<br>$Jb_F$=3.6<br>$Jd_F$=3.2 | 1680<br>(1640) | |
| $CH_3^b$ | $C_6H_5^c$ | a)c) 7.0~7.6<br>E b) 1.96 (d)<br>Z b) 2.24 (d) | 43.7 (q)<br>46.2 (q) | $Jb_F$=4.5<br>$Jb_F$=3.6 | 1680, 1685<br>(1630, 1640) | E:114~115<br>64~70<br>(E/Z=35/65) |
| $CH_3^b$ | $(CH_3^c)_2CH^d$ | E { a) 7.33 (s)<br>b) 1.71 (d)<br>c) 1.02 (d)<br>d) 3.76 (d, sep) }<br>Z { b) 1.91 (d)<br>c) 1.02 (d)<br>d) 3.08 (d, sep) } | 46.8 (br)<br>49.0 (q) | Jcd=7.1<br>$Jb_F$=4.5<br>$Jd_F$=3.0<br>$Jb_F$=3.8<br>$Jd_F$=1.1 | 1685, 1690<br>(1635) | |
| $CH_3^bCH_2^c$ | $H^d$(Z) | a) 7.39 (s)<br>b) 1.05 (t)<br>c) 2.22 (d, d, q)<br>d) 5.99 (d, t) | 51.0 (d, t) | Jbc=7.5<br>Jcd=7.5<br>$Jc_F$=2.3<br>$Jd_F$=35.0 | 1685<br>(1660) | |
| $CH_3^bCH_2^c$ | $CH_3^dCH_2^e$ | a) 7.39 (s)<br>b) 1.08 (t)<br>c) 2.47 (d, q)<br>d) 1.00 (t)<br>e) 2.22 (d, q) | 50.0 (br) | Jbc=Jde=7.5<br>$Jc_F$=1.5<br>$Je_F$=3.5 | 1675<br>(1635) | |
| $CH_3^bCH_2^c$ | $C_6H_5^d$(E) | a) 7.39 (s)<br>b) 1.02 (t)<br>c) 2.56 (d, q)<br>d) 7.1~7.4 | 46.0 (t) | Jbc=7.5<br>$Jc_F$=3.8 | 1680<br>(1630) | 116~117 |

TABLE 9′

| $R^1R^3C{=}CF^{b'}CF_2^{a'}SC_6H_5$ | | NMR | | |
|---|---|---|---|---|
| | | Chem. shift (δ ppm) | | Coupling const. (Hz) |
| $R^1$ | $R^3$ | $^1H$ (solv.: $CCl_4$) | $^{19}F$ (no solv.) | |
| $CH_3^a$ | $CH_3^b$ | 1.59 (d, d)<br>1.68 (d, d)<br>Ph) o, p 7.2—7.5<br>m 7.5—7.7 | 46.0 (t, sep)<br>−4.8 (d, sep) | Ja—a′=2.8, Ja′—b′=5.4<br>Ja—b′=2.9, Jb—b′=4.2<br>Jb—a′=2.6 |
| $CH_3^a$ | $C_6H_5$ | E and Z<br>1.8~2.0 ($CH_3$, m)<br>7.0~7.7 (Ar-H) | E a′)−5.7<br>b′) 41.8<br>Z a′) −4.7<br>b′) 43.0 | E Ja—a′=1.9, Ja—b′=4.1<br>$J_{F-F2}$=18.8<br>Z $J_{F-F}$=15.1<br>Ja—b′=3.8<br>Ja—a′=2.8 |

TABLE 10

| $R^1$ | $R^3$ | Yield* (%) | E/Z Ratio | B.p. (°C/mmHg) |
|---|---|---|---|---|
| $CH_3$— | $CH_3$— | 36 (85) | — | 62~72/48 |
| $CH_3$— | i-$C_3H_7$— | 57 (82) | 32/68 | 70~73/21 |
| $CH_3$— | Ph— | 81 (97) | 11/89 | — |

* Isolation yield; $^{19}F$ NMR yield in parentheses.
(External standard: $PhCF_3$).

The above thioester *6* can also be converted to some useful fluorinated compounds other than the allyl alcohol *8* as mentioned above.

For example, if reacted with an alkoxide, it readily undergoes an ester interchange according to the following reaction to give the corresponding ester *9* in high yield.

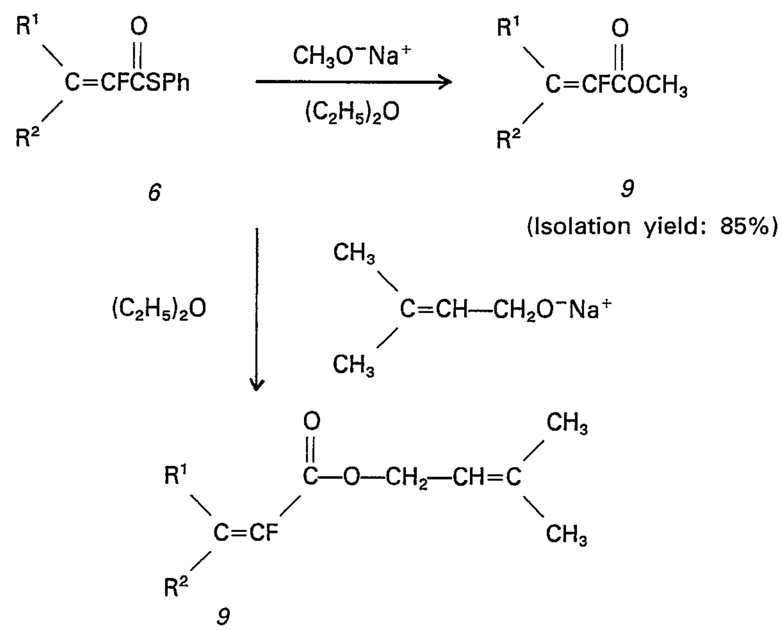


6

9

(Isolation yield: 85%)

9

(Isolation yield: 80%)

TABLE 11

| $R^3CH_3{}^aC{=}CFCH_2{}^bOH^c$ | NMR | | | IR |
|---|---|---|---|---|
| | Chem. shift (δ ppm) | | Coupling const. (Hz) | $\nu_{O-H}$ ($cm^{-1}$) |
| | $^1H$ (solv.: $CCl_4$) | $^{19}F$ (—) | | |
| $R^3{=}(CH_3{}^d)_2CH^e$ | a) 1.60 (d)<br>c) 3.55 (br)<br>d) 1.05 (d)<br>E{b) 4.09 (d)<br>E{e) 2.67 (d, sep)<br>Z{b) 4.11 (d)<br>Z{e) 3.00 (sep) | 38.7 (t)<br>39.8 (t) | $Ja_F=2.4$<br>$Jb_F=23.2$<br>$Jde=6.8$<br>$Je_F(E)=3.0$ | 3350 |
| $R^3{=}C_6H_5{}^d$ | a) 1.96 (d)<br>c) 3.09 (br)<br>d) 7.25 (s)<br>E:b) 4.26 (d)<br>Z:b) 4.00 (d) | 36.3 (t)<br>37.0 (t) | $Ja_F=3.0$<br>$Jb_F=23.1$ | 3400 |

Further, having an unsaturated bond in its molecule, the thioester *6* can be converted to the corresponding cyclic compound, and particularly heterocyclic compound.

To further illustrate this invention, and not by way of limitation, the following examples are given. It is noted that in these examples the $^1H$ NMR data was determined in solvent $CCl_4$ with reference to the internal standard tetramethylsilane while $^{19}F$ NMR data was determined with reference to the external standard trifluoroacetic acid without use of any solvent.

[Examples]

Synthesis of 4-phenylthio-3,4,4-trifluoro-2-butanone

8.03 g (50 mmol) of 4 - chloro - 3,4,4 - trifluoro - 2 - butanone, 5.51 g (50 mmol) of thiophenol and 70 ml of methylene chloride were put in a 200 ml round-bottomed flask. With the flask in ice bath for cooling, 5.06 g (50 mmol) of triethylamine diluted with 30 ml of methylene chloride was added dropwise. After addition, the ice bath was removed and the mixture was agitated half an hour at room temperature. Water was then added and the methylene chloride fraction was separated, which was washed once with 1N HCl, a few times with water, and dried on anhydrous magnesium sulfate. Evaporation of methylene chloride was followed by distillation to obtain 11.17 g (48 mmol) of 4 - phenylthio - 3,4,4 - trifluoro - 2 - butanone $CH_3C(O)CHFCF_2SPh$ in the yield of 96%. Its boiling point was 78 to 80°C/0.6 mmHg and its parent peak in mass spectrogram was found at 234. Its NMR and IR spectral data were as already mentioned.

Synthesis of 5-phenylthio-4,5,5-trifluoro-3-pentanone

The foregoing procedure was conformed to for the synthesis. The mass spectroscopy of the product gave the parent peak at 248.

Synthesis of 1,2-difluoro-1-phenylthio-1-buten-3-one

7.02 g (30.0 mmol) of 4 - phenylthio - 3,4,4 - trifluoro - 2 - butanone and 50 ml of 2-propanol were put in a 100 ml round-bottomed flask and with the flask in ice bath for cooling 3.03 g (30 mmol) of triethylamine was added. The ice bath was removed and the solution was agitated 3 hr at room temperature. The reaction solution was then poured into water and ether was used for extraction. The ether fraction was washed once with 1N HCl, twice with water and then dried on anhydrous magnesium sulfate. Evaporation of the solvent was followed by distillation to obtain 6.14 g (28.7 mmol) of 1,2 - difluoro - 1 - phenylthio - 1 - buten - 3 - one in the yield of 96%. Its boiling point was 81 to 95°C/0.2 mmHg (E/Z ratio=2/3) and its parent peak in mass spectrogram was found at 214. Its NMR and IR spectral data were as already mentioned.

Synthesis of 1,2-difluoro-1-phenylthio-1-penten-3-one

The foregoing procedure was conformed to for the synthesis. The mass spectroscopy of the product gave the parent peak at 228.

[Applications]

Reaction of 1,2 - difluoro - 1 - phenylthio - 1 - buten - 3 - one and 1,2 - difluoro - 1 - phenylthio - 1 - penten - 3 - one with Grignard reagent

Only the reaction of 1,2 - difluoro - 1 - phenylthio - 1 - buten - 3 - one with methylmagnesium iodide is described here by way of example though similar experiments were performed with the other compound.

Methylmagnesium iodide was prepared using 4.26 g (30 mmol) of iodomethane, 0.80 g (33 mg-atom) of metallic magnesium, and 50 ml of dry ether. With the flask in ice bath for cooling, 4.28 g (20.0 mmol) of 1,2 - difluoro - 1 - phenylthio - 1 - buten - 3 - one diluted with 10 ml of ether was slowly added thereto, drop by drop. After addition, the solution was agitated half an hour while being cooled in ice bath. The reaction solution was then poured into saturated ammonium chloride solution, from which extraction was made using ether. The ether fraction was dried on anhydrous magnesium sulfate and ether was removed by evaporation. The residues were column chromatographed using 1% triethylamine solution in n-hexane-ether mixture as the developing solvent system to isolate 4.51 g (19.6 mmol) of the carbinol $(CH_3)_2C(OH)CF{=}CFSPh$ (yield: 98%).

Synthesis of α-fluoro-α,β-unsaturated carboxylic acid thioester

Only the synthesis of the compound $CH_3CH_2C(CH_3){=}CFC(O)SPh$ is described here by way of example though similar experiments were performed for the synthesis of similar compounds. Either or both of the two aforementioned methods, namely, the heating method and method of adding conc. sulfuric acid were used.

Method (1): 2.44 g (10.0 mmol) of the carbinol EtMeC(OH)CF=CFSPh was put in a 50 ml round-bottomed flask. After the flask was fitted with an air condenser tube, the compound was heated to 120 to 140°C on oil bath without addition of any solvent (as the reaction proceeded, HF was generated and the solution turned black after the reaction was completed in several seconds). 3.0 g (30 mmol) of triethylamine and 30 ml of n-hexane were added to the solution. After filtering out salt precipitates, the filtrate was dried on anhydrous magnesium sulfate. N-hexane was evaporated and the residues were column chromatographed making use of the 1% triethylamine solution in n-hexane-ether mixture as the developing solvent system to isolate 1.91 g (8.5 mmol) of pure EtMeC=CFC(O)SPh (yield: 85%, E/Z ratio=31/69).

Method (2): 2.44 g (10.0 mmol) of the carbinol EtMeC(OH)CF=CFSPh was put in a 50 ml round-bottomed flask.

A drop of conc. sulfuric acid was dropped in the flask without addition of any solvent. The reaction was completed immediately with generation of hydrogen fluoride turning the compound black. 3.0 g (30 mmol) of triethylamine and 30 ml of n-hexane were added thereto. After filtering out salt precipitates, the filtrate was dried on anhydrous magnesium sulfate. N-hexane was evaporated and the residues were column chromatographed to isolate 1.57 g (7.0 mmol) of pure EtMeC=CFC(O)SPh (yield: 70%, E/Z ratio: 39/61). The related NMR and IR spectral data was as already mentioned. The mass spectroscopy gave parent peaks of the above and similar compounds obtained as follows.

$Me_2C{=}CFC(O)SPh$: 210,
PhMeC=CFC(O)SPh: 272,
EtMeC=CFC(O)SPh: 224,
i-PrMeC=CFC(O)SPh: 238,
i-PrMeC(OH)CF=C(SPh)i-Pr: 264, and
$PhMeC{=}CFCF_2SPh$: 294.

Reduction of 1,2-difluoro-1-phenylthio-1-buten-3-one with sodium boron hydride

0.19 g (5 mmol) of $NaBH_4$ and 10 ml of ethanol were put in a 50 ml round-bottomed flask. With the flask in ice bath for cooling, 2.14 g (10.0 mmol) of 1,2 - difluoro - 1 - phenylthio - 1 - buten - 3 - one was added. The solution was agitated 1 hr with the flask still in the ice bath and 1N HCl was then added to stop the reaction. After extraction with ether, the ether fraction was dried on anhydrous magnesium sulfate and the solvent was evaporated. The residues were column chromatographed using 1% triethylamine solution in n-hexane-ether mixture as the developing solvent system to isolate 2.12 g (9.8 mmol) of $CH_3CH(OH)CF{=}CFSPh$ (yield: 98%).

Reduction of 1,2-difluoro-1-phenylthio-1-penten-3-one with sodium boron hydride

The foregoing procedure was conformed to.

Synthesis of methyl 2-fluorosenecionate $Me_2C{=}CFCO_2Me$

Sodium methoxide was prepared using 0.29 g (12 mmol) of sodium hydride, 0.38 g (12 mmol) of dry methanol and 20 ml of dry ether. While cooling in ice bath, 2.10 g (10.0 mmol) of 2-fluorosenecionic acid thioester ($Me_2C{=}CFC(O)SPh$) diluted with 5 ml of dry ether was added dropwise and then the ice bath was removed. After 1 hr agitation at room temperature, the solution was poured into 1N HCl for extraction with ether. The ether fraction was dried on anhydrous magnesium sulfate and the solvent ether was evaporated. The residues were column chromatographed to isolate 1.12 g (8.5 mmol) of pure methyl 2-fluorosenecionate (yield: 85%).

Synthesis of pulenyl 2-fluorosenecionate ($Me_2C{=}CFCO_2CH_2CH{=}CMe_2$)

The foregoing procedure was conformed to.

Reduction of α-fluoro-α,β-unsaturated carboxylic acid thioester with sodium boron hydride

Only the case of 2-fluorosenecionic acid thioester is described here by way of example though similar experiments were performed with other two similar compounds of the invention.

0.45 g (12 mmol) of sodium boron hydride and 10 ml of ethanol were put in a 50 ml round-bottomed flask. With the flask in ice bath for cooling, 2.10 g (10.0 mmol) of the compound $Me_2C{=}CFC(O)SPh$ was added. After 2 hr agitation, 1N HCl was added to stop the reaction. Extraction was made with ether and the ether fraction was dried on anhydrous magnesium sulfate. After evaporation of the solvent ether, the residues were distilled to obtain 0.37 g (3.6 mmol) of $(CH_3)_2C{=}CFCH_2OH$ (b.p.: 69—72°C/48 mmHg, yield: 36%). The related NMR spectral data was already mentioned. The IR($\nu_{O-H}$) spectral data was as follows.

$Me(i\text{-}Pr)C{=}CFCH_2OH$: 3350 ($cm^{-1}$),
$PhMeC{=}CFCH_2OH$: 3400 ($cm^{-1}$), and
$Me_2C{=}CFCH_2OH$: 3350 ($cm^{-1}$).

Reaction of 1,2-difluoro-1-phenylthio-1-buten-3-one with enolate

Only the case of the enolate of acetone is described here by way of example though a similar experiment was performed with the enolate of acetophenone.

Lithium N,N-diisopropylamide was prepared from 4.2 ml (3.03 g, 30 mmol) of diisopropylamine and 18.0 ml of 1.6M n-BuLi/hexane. 40 ml of dry tetrahydrofuran was added thereto. With the flask in dry ice bath for cooling at −78°C, 2.20 ml (1.73 g, 30 mmol) of dry acetone was added slowly and the solution was agitated half an hour at this temperature as it was. 4.28 g (20.0 mmol) of 1,2 - difluoro - 1 - phenylthio - 1 - buten - 3 - one diluted with 5 ml of dry tetrahydrofuran was slowly added to this solution at −78°C. After 5 hr agitation with the flask still in the dry ice bath, 5.0 ml (4.3 g, 40 mmol) of chlorotrimethylsilane was added. The solution was then removed from the dry ice bath. After recovery to room temperature, the solution was agitated half an hour. Salt precipitates were removed from the reaction solution by filtration under suction. Solvent was evaporated from the filtrate and the residues were column chromatographed using 1% triethylamine solution in n-hexane-ether mixture as the developing solvent system to isolate 6.02 g (17.5 mmol) of pure compound

$CH_3C(O)CH_2C(OSiMe_3)CH_3CF{=}CFSPh$ (yield: 88%).

Reaction of 1,2-difluoro-1-phenylthio-1-buten-3-one with sodium diethyl malonate

1.06 g (44 mmol) of sodium hydride and 40 ml of dry tetrahydrofuran were put in a 100 ml three necked flask. With the flask in ice bath for cooling, 6.70 ml (7.05 g, 44 mmol) of diethyl malonate was added to prepare sodium diethyl malonate. With the flask still in the ice bath, 4.28 g (20.0 mmol) of the compound $CH_3C(O)CF{=}CFSPh$ diluted with 10 ml of dry tetrahydrofuran was slowly added and the solution was agitated 3 hr as it was. The reaction solution was poured in 1N HCl and extraction was made with ether. The ether fraction was dried on anhydrous magnesium sulfate and the solvent was evaporated. The residues were column chromatographed to isolate 6.36 g (18.0 mmol) of pure compound

$CH_3C(O)CF{=}C(SPh)CH(CO_2Et)_2$ (yield: 90%).

Reaction of 1,2-difluoro-1-phenylthio-1-buten-3-one with lithium diethyl malonate

Lithium N,N-diisopropylamide was prepared from 10 ml of dry hexane, 4.5 ml (3.25 g, 32 mmol) of diisopropylamine and 19.0 ml of 1.6 M n-BuLi/hexane. By solvent exchange, 30 ml of dry tetrahydrofuran was introduced. With the flask in the ice bath, 4.60 ml (4.85 g, 30 mmol) of diethyl malonate was added for the synthesis of lithium diethyl malonate. 4.28 g (20.0 mmol) of the compound $CH_3C(O)CF{=}CFSPh$ diluted with 5 ml of dry tetrahydrofuran was added thereto. The solution was agitated 3 hr with the flask still in ice bath. The reaction solution was poured in 1N HCl and extraction was made with ether. The ether fraction was dried on anhydrous magnesium sulfate and the solvent was evaporated. The residues were column chromatographed to isolate 4.94 g (13.9 mmol, yield: 70%) of the compound

$CH_3C(O)CF{=}C(SPh)CH(CO_2Et)_2$.

The unreacted compound $CH_3C(O)CF{=}CFSPh$ that could be recovered after reaction amounted to 0.66 g (3.1 mmol, 16% in yield, E form only).

Reaction of 1,2-difluoro-1-phenylthio-1-buten-3-one with thiophenol

20 ml of 2-propanol, 2.20 g (20.0 mmol) of thiophenol, and 4.28 g (20.0 mmol) of the compound $CH_3C(O)CF{=}CFSPh$ were put in a 50 ml round-bottomed flask and 3.04 g (30 mmol) of triethylamine was added slowly at room temperature. After half an hour agitation at room temperature, the solution was poured in water and extraction was performed with ether. The ether fraction was washed with 1N HCl and then with water and dried on anhydrous magnesium sulfate. After evaporation of the solvent, the residues were recrystallized from n-hexane-ether mixture (10:1) to isolate 4.69 g (15.4 mmol) of pure 1,1 - bis(phenylthio) - 2 - fluoro - 1 - buten - 3 - one (yield: 77%, m.p.: 72—75°C).

Reaction of 1,2-difluoro-1-phenylthio-1-buten-3-one with n-propylamine

Only the reaction with n-propylamine is described here though a similar experiment was made with diethylamine.

4.28 g (20 mmol) of the compound $CH_3C(O)CF{=}CFSPh$ and 20 ml of 2-propanol were put in a 50 ml round-bottomed flask. With the flask in ice bath for cooling, 2.60 g (44 mmol) of n-propylamine was slowly added and the solution was agitated half an hour as it was. The reaction solution was poured in 5% sodium bicarbonate solution and extraction was performed with ether. The ether fraction was dried on anhydrous magnesium sulfate and solvent was evaporated. The residues were column chromatographed using 1% triethylamine solution in n-hexane-ether mixture as the developing solvent system to isolate 4.85 g (19.1 mmol) of the pure compound

$$CH_3C(O)CF{=}C(SPh)NH(n\text{-}Pr).$$

The product comprised a mixture of the enamine and imine forms accounting for 75% and 25% thereof, respectively.

Reaction of 1,2-difluoro-1-phenylthio-1-buten-3-one with methanol

4.28 g (20.0 mmol) of the compound $CH_3C(O)CF{=}CFSPh$, 20 ml of methanol, and 3.04 g (30 mmol) of triethylamine were put in a 50 ml round-bottomed flask. The flask was fitted with a Dimroth condenser and the solution was refluxed 5 hr. After recovery to room solution, the reaction solution was poured in water and extraction was performed with ether. The ether fraction was washed with 1N HCl and then with water and dried on anhydrous magnesium sulfate. After evaporation of solvent, the residues were column chromatographed using 2% triethylamine solution in n-hexane-ether mixture as the developing solvent system to isolate 2.34 g (10.4 mmol) of the pure compound

$$CH_3C(O)CF{=}C(OMe)SPh \text{ (yield: 52\%).}$$

Products of the above reactions are listed in the following table.

TABLE 12

| | NMR | | | IR ($cm^{-1}$) |
|---|---|---|---|---|
| | Chem. shift (δ ppm) | | Coupling const. (Hz) | |
| | $^{1}H$ (solv.: $CCl_4$) | $^{19}F$ (no solv.) | | |
| 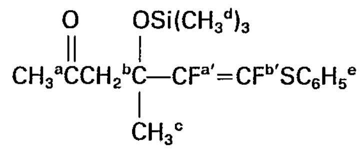 | a) 1.94 (s), 2.03 (s)<br>b) 2.80 (d), 2.87 (d)<br>c) 1.56 (d), 1.60 (d)<br>d) 0.10 (s), 0.11 (s)<br>e) 7.1~7.6 | E {a') 36.1 (br), b') 31.9 (d)}<br>Z {a') 53.5 (d), b') 46.7 (d)} | Jca'=5.0<br>Ja'b'= {E 3.8, Z 141.0} | 1720<br>($v_c$=0) |
| 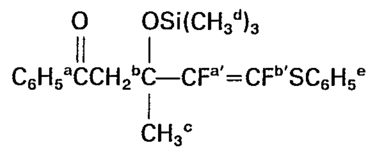 | a) {o, p 7.1~7.6, m 7.7~8.1}<br>b) 3.45 (d)<br>c) 1.70 (d), 1.75 (d)<br>d) 0.10 (s)<br>e) 7.1~7.6 | E {a') 35.3 (br), b') 31.7 (d)}<br>Z {a') 53.0 (d), b') 46.7 (d)} | Jca'=5.4<br>Ja'b'= {E 4.0, Z 143.0} | 1685<br>($v_c$=0) |
| 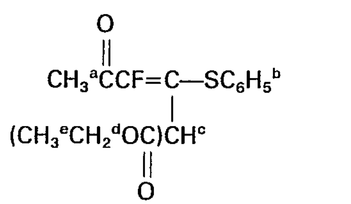 | a) {E 2.22 (d), Z 2.32 (d)}<br>b) 7.2~7.6<br>c) 5.28 (s)<br>d) 4.20 (q)<br>e) 1.30 (t) | E 21.8 (q)<br>Z 32.1 (q) | $Ja_F$=5.4<br>Jde=7.2 | 1695, 1740<br>($v_c$=0)<br>1580 ($v_c$=c) |
| 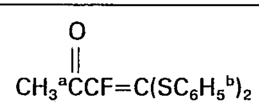 | a) 2.28 (d)<br>b) 6.9~7.2 | 21.0 (q)<br>(Solv.: $Et_2O$) | $Ja_F$=4.5 | 1675 ($v_c$=0) |

TABLE 12 (contd.)

| | NMR | | | |
|---|---|---|---|---|
| | Chem. shift (δ ppm) | | Coupling const. (Hz) | IR ($cm^{-1}$) |
| | $^1H$ (solv.: $CCl_4$) | $^{19}F$ (no solv.) | | |
| 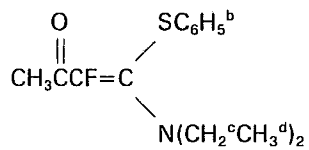 (E/Z=46/54) | a) {E 2.23 (d), Z 2.11 (d)}<br>b) 7.1~7.5<br>c) 3.14 (q)<br>d) {E 1.00 (t), Z 0.94 (t)} | E: 49.6 (br)<br>Z: 60.3 (q) | Jcd=7.5<br>$Ja_F$={4.8 (E), 5.9 (Z)} | 1660 ($\nu_c$=0)<br>1540 ($\nu_c$=c) |
| 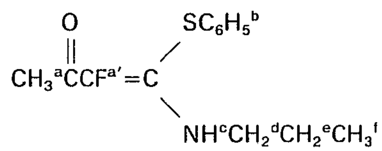<br>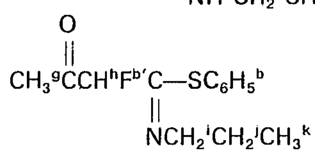<br>Enamine Imine<br>(E/Z=50/50):(E/Z=<1/>99)<br>=75:25 | a) 2.13 (d) g) 2.01 (d)<br>b) 7.2~7.6 h) 4.78 (d)<br>c) 9.5 (br) i) 3.42 (t)<br>d) 3.23 (t) j) 1.72 (t, q)<br>e) 1.48 (t, q) k) 0.97 (t)<br>f) 0.89 (t) | a') 78.8 (q)<br>79.0 (q)<br>b') 96.5 (d, q) | Jde=7.5<br>Jij=7.4<br>Jaa'=4.7<br>Jgb'=4.0<br>Jhb'=48.9 | Enamine<br>1620 ($\nu_c$=0)<br>1560 ($\nu_c$=c)<br>3200 ($\nu_{N-H}$)<br>Imine<br>1745, 1725<br>($\nu_c$=0 c=N) |
| 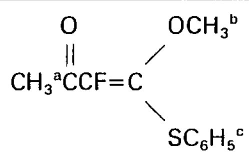 | a) 2.29 (d)<br>b) 3.61 (s)<br>c) 7.2~7.6 | 62.1 (q) | $Ja_F$=3.0 | 1675 ($\nu_c$=0)<br>1570 ($\nu_c$=c) |

**Claims**

1. A sulfur-containing fluorinated unsaturated compound having the general formula

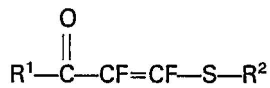

wherein $R^1$ and $R^2$ are independently alkyl groups having up to eight carbon atoms with or without a substituent or substituents and $R^2$ can also be a phenyl group with or without a substituent or substituents.

2. A sulfur-containing fluorinated unsaturated compound having the general formula

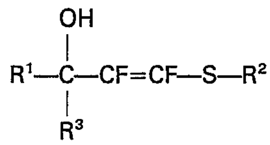

wherein $R^1$, $R^2$ and $R^3$ are independently alkyl groups having up to eight carbon atoms with or without a substituent or substituents and $R^2$ and $R^3$ can also be phenyl groups with or without a substituent or substituents.

3. A process for the manufacture of a sulfur-containing fluorinated unsaturated compound characterized in that a halide of a fluorinated carbonyl compound having the general formula

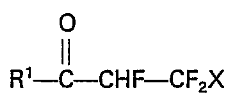

wherein $R^1$ is an alkyl group having up to eight carbon atoms with or without a substituent or substituents and X is a halogen atom, is reacted with a mercaptan having the general formula

$$R^2\text{—}SH$$

wherein $R^2$ is an alkyl group having up to eight carbon atoms or phenyl group each with or without a substituent or substituents, to produce a sulfur-containing fluorinated carbonyl compound having the general formula

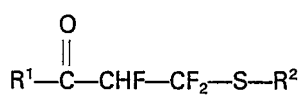

wherein $R^1$ and $R^2$ are the same as those of the starting compounds and said sulfur-containing fluorinated carbonyl compound is converted, by the elimination of hydrogen fluoride, to the corresponding sulfur-containing fluorinated unsaturated carbonyl compound having the general formula

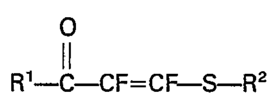

wherein $R^1$ and $R^2$ are the same as those of the starting compounds.

4. The process as claimed in claim 3, characterized in that said halide of fluorinated carbonyl compound is reacted with said mercaptan in the presence of a base.

5. The process as claimed in claim 3, characterized in that said hydrogen fluoride is eliminated from said sulfur-containing fluorinated carbonyl compound in an alcoholic solvent under the action of a base.

**Patentansprüche**

1. Schwefelhaltige fluorierte ungesättigte Verbindung der allgemeinen Formel

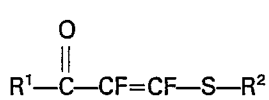

worin $R^1$ und $R^2$ voneinander unabhängig Alkylgruppen mit bis zu 8 Kohlenstoffatomen mit oder ohne

Substituent oder Substituenten bedeuten und $R^2$ ebenfalls eine Phenylgruppe mit oder ohne Substituent oder Substituenten sein kann.

2. Schwefelhaltige fluorierte ungesättigte Verbindung der allgemeinen Formel

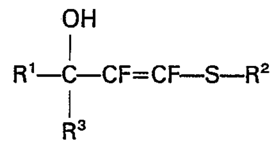

worin $R^1$, $R^2$ und $R^3$ voneinander unabhängig Alkylgruppen mit bis zu 8 Kohlenstoffatomen mit oder ohne Substituent oder Substituenten bedeuten und $R^2$ und $R^3$ ebenfalls Phenylgruppen mit oder ohne Substituent oder Substituenten sein können.

3. Verfahren zur Herstellung einer schwefelhaltigen fluorierten ungesättigten Verbindung, dadurch gekennzeichnet, daß man ein Halogenid einer fluorierten Carbonylverbindung der allgemeinen Formel

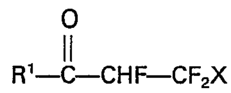

worin $R^1$ eine Alkylgruppe mit bis zu 8 Kohlenstoffatomen mit oder ohne einem Substituent oder Substituenten und X ein Halogenatom bedeuten, mit einem Mercaptan der allgemeinen Formel

$$R^2—SH$$

worin $R^2$ eine Alkylgruppe mit bis zu 8 Kohlenstoffatomen oder eine Phenylgruppe jeweils mit oder ohne Substituent oder Substituenten bedeuten, unter Bildung einer schwefelhaltigen fluorierten Carbonylverbindung der allgemeinen Formel

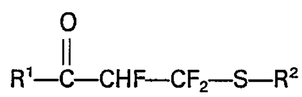

worin $R^1$ und $R^2$ die Bedeutungen der Ausgangsverbindungen haben und umsetzt, die schwefelhaltige fluorierte Carbonylverbindung durch Entfernen von Fluorwasserstoff in die entsprechende schwefelhaltige fluorierte ungesättigte Carbonylverbindung der allgemeinen Formel

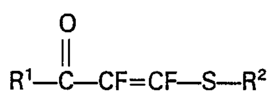

worin $R^1$ und $R^2$ die Bedeutungen der Ausgangsverbindungen haben, umwandelt.

4. Verfahren nach Anspruch 3, dadurch gekennzeichnet, daß man das Halogenid der fluorierten Carbonylverbindung mit dem Mercaptan in Gegenwart einer Base umsetzt.

5. Verfahren nach Anspruch 3, dadurch gekennzeichnet, daß man den Fluorwasserstoff aus der schwefelhaltigen fluorierten Carbonylverbindung in einem alkoholischen Lösungsmittel unter Mitwirkung einer Base entfernt.

**Revendications**

1. Composé insaturé fluoré contenant du soufre, de formule générale:

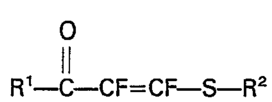

dans laquelle $R^1$ et $R^2$ sont, indépendamment, des groupes alkyle ayant jusqu'à 8 atomes de carbone, non substitués ou comportant un ou des substituants, et $R^2$ peut également être un groupe phényle non substitué ou comportant un ou des substituants.

2. Composé insaturé fluoré contenant du soufre, de formule générale:

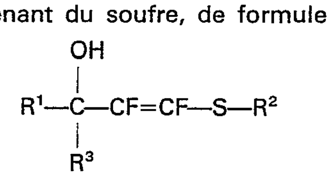

dans laquelle $R^1$, $R^2$ et $R^3$ sont, indépendamment, des groupes alkyle ayant jusqu'à 8 atomes de carbone, non substitués ou comportant un ou des substituants, et $R^2$ et $R^3$ peuvent également être des groupes phényle non substitué ou comportant un ou des substituants.

3. Procédé pour la préparation d'un composé insaturé fluoré contenant du soufre, caractérisé en ce que l'on fait réagir un halogénure d'un composé carbonylé fluoré de formule générale:

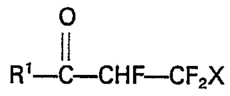

dans laquelle $R^1$ est un groupe alkyle ayant jusqu'à 8 atomes de carbone, non substitué ou comportant un ou des substituants, et X est un atome d'halogène, avec un mercaptant de formule générale:

$$R^2\text{—SH}$$

dans laquelle $R^2$ est un groupe alkyle ayant jusqu'à 8 atomes de carbone, ou un groupe phényle, non substitués ou comportant un ou des substituants, pour obtenir un composé carbonylé fluoré contenant du soufre, de formule générale:

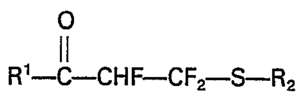

dans laquelle $R^1$ et $R^2$ sont les mêmes radicaux que ceux des composés de départ, et on convertit ledit composé carbonylé fluoré contenant du soufre, par l'élimination d'acide fluorhydrique, en le composé carbonylé fluoré insaturé correspondant, contenant du soufre, de formule générale:

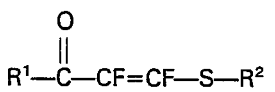

dans laquelle $R^1$ et $R^2$ sont les mêmes radicaux que ceux des composés de départ.

4. Procédé selon la revendication 3, caractérisé en ce que l'on fait réagir ledit halogénure du composé carbonylé fluoré avec ledit mercaptan en présence d'une base.

5. Procédé selon la revendication 3, caractérisé en ce que ledit acide fluorhydrique est éliminé hors dudit composé carbonylé fluoré contenant du soufre, dans un solvant alcoolique, sous l'action d'une base.